# EUROPEAN PATENT APPLICATION

(11) **EP 0 794 006 A1**
(43) Date of publication of application: **10.09.1997**
(21) Application number: 97103753.6
(22) Date of filing: 06.03.1997
(51) Int. Cl.: B01J 37/00, C07C 29/153

(54) **Metal-metal oxide catalyst and method for production thereof by mechanical alloying treatment**

(30) Priority: 08.03.1996 JP 79382/96
(71) Applicant: YKK CORPORATION, Chiyoda-ku, Tokyo (JP); AGENCY OF INDUSTRIAL SCIENCE AND TECHNOLOGY, Tokyo (JP)
(72) Inventor: Yamaguchi, Tadashi, Sendai-shi, Miyagi-ken (JP); Fukui, Hideo, Sendai-shi, Miyagi-ken (JP); Kobayashi, Masayuki, Kurokawa-gun, Miyagi-ken (JP); Okabe, Kiyomi, Tsukuba-shi, Ibaraki-ken (JP); Kusama, Hitoshi, Tsukuba-shi, Ibaraki-ken (JP); Arakawa, Hironori, Tsukuba-shi, Ibaraki-ken (JP); Sayama, Kazuhiro, Tsukuba-shi, Ibaraki-ken (JP)
(74) Representative: Patentanwälte Leinweber & Zimmermann

(57) **Abstract**

Disclosed are a metal-metal oxide composite catalyst which has the catalytic structure thereof altered by a mechanical alloying treatment to the structure sparingly inducible of sintering and a method for the production thereof. The composite catalyst comprises catalyst particles or microporous catalyst particles each having a plurality of catalytically active metal particles and metal oxide particles mutually bonded in a dispersed state. Such a metal-metal oxide catalyst is produced by subjecting a mixture of a catalytically active metal and a metal oxide, severally in the form of a powder, granules, or foils, to the mechanical alloying treatment. The catalyst of the microporous structure is produced by subjecting a mixture of a catalytically active metal, a metal oxide, and a metal soluble in a developing solution, severally in the form of a powder, granules, or foils, to the mechanical alloying treatment and then performing a developing treatment on the produced particles by the use of the developing solution thereby dissolving out the soluble metal from the particles.

## Description

### BACKGROUND OF THE INVENTION

### 1. Field of the Invention:

This invention relates to a metal-metal oxide catalyst and a method for the production thereof by a mechanical alloying treatment, and more particularly to a catalyst for hydrogenation of carbon dioxide and/or carbon monoxide to be used in the synthesis of an alcohol and/or a hydrocarbon by the reaction of carbon dioxide, carbon monoxide, or the mixture of carbon dioxide and carbon monoxide with hydrogen gas, and to a method for the production of the catalyst.

### 2. Description of the Prior Art:

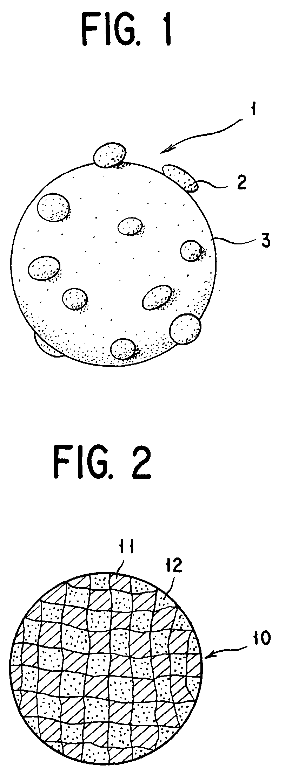

The heretofore predominant methods for the production of catalysts are coprecipitation method, impregnation method, and other similar methods which use metal salt solutions as starting raw materials. The coprecipitation method consists in causing a metal salt solution to react with an alkali to produce a metal hydroxide and converting the metal hydroxide by calcining into a metal oxide. The impregnation method consists in impregnating a porous ceramic substance with a metal salt and calcining the impregnated porous ceramic substance thereby producing a metal oxide. Optionally, the obtained metal oxide is further subjected, immediately prior to use, to a pretreatment in a reducing atmosphere such as hydrogen thereby producing, as illustrated in Fig. 1, a catalyst 1 having minute particles 2 of a catalytically active metal deposited fast as dispersed like islands on the surface of a porous ceramic carrier 3.

Though the catalyst produced as described above is highly active, it has the problem of readily incurring deactivation, i.e. insufficient durability of the catalyst, because the catalyst particles are bound or agglomerated by sintering or the problem of imposing a limit on the combination of the component elements of the raw material. The conventional method for the production of a catalyst also has the problem of necessitating many steps for the process and, as a result, complicating the process. Further, the catalyst produced by the conventional method must be given, immediately prior to use, a pretreatment in such a reducing atmosphere as hydrogen. If the procedure of this treatment happens to involve an error, the catalyst will suddenly emit heat and sinter and ultimately cease to manifest the activity inherent therein. Thus, the treatment is in need of accurate control of temperature. The catalyst, therefore, has the problem of calling for a complicated pretreatment prior to use.

### SUMMARY OF THE INVENTION

A primary object of the present invention is to provide a metal-metal oxide composite catalyst which has the catalytic structure thereof altered by a mechanical alloying treatment to the structure sparingly inducible of sintering, and which consequently excels in the durability (active durability) and in the catalytic activity.

A further object of the present invention is to provide a metal-metal oxide microporous catalyst which has the catalytic activity thereof enhanced by causing the particles of the catalyst to acquire a microporous structure sparingly inducible of sintering.

Another object of the present invention is to provide a method for the production of such a metal-metal oxide catalyst as mentioned above, which method produces the catalyst by a simplified process as compared with the conventional method using a metal salt solution as the starting raw material and obviates the necessity for an operationally complicated reducing pretreatment.

To accomplish the objects mentioned above, one aspect of the present invention provides a composite catalyst comprising a metal and a metal oxide. One embodiment thereof is characterized by comprising particles each having a plurality of catalytically active metal particles and metal oxide particles mutually bonded in a dispersed state. The metal-metal oxide catalyst of another embodiment of the present invention is characterized by comprising microporous particles each having a plurality of catalytically active metal particles and metal oxide particles mutually bonded in a dispersed state.

In a preferred embodiment of the invention, at least one metal selected from the group consisting of Fe, Co, Ni, Cu, Rh, Pd, Ag, Pt, and Au is used as the catalytically active metal mentioned above and at least one metal oxide selected from the group consisting of the oxides of Mg, Al, Si, P, Ca, Sc, Ti, V, Cr, Mn, Fe, Co, Ni, Cu, Zn, Ga, Ge, Sr, Y, Zr, Nb, Mo, Cd, In, Sn, Ba, Hf, Ta, W, Pb, Bi, La, Ce, Pr, Nd, Pm, Sm, Eu, Gd, Tb, Dy, Ho, Er, Tm, Yb, and Lu is used as the metal oxide mentioned above.

Further the present invention provides a method for the production of such a metal-metal oxide catalyst as mentioned above. One embodiment thereof is characterized by subjecting a mixture of a catalytically active metal and a metal oxide, severally in the form of a powder, granules, or foils, to a mechanical alloying treatment thereby forming catalyst particles each having a plurality of catalytically active metal particles and metal oxide particles mutually bonded in a dispersed state.

The method for the production of a metal-metal oxide catalyst as another embodiment is characterized by comprising a step of subjecting a mixture of a catalytically active metal, a metal oxide, and a metal soluble in a developing solution, severally in the form of a powder, granules, or foils, to a mechanical alloying treatment, thereby producing particles each having a plurality of catalytically active metal particles, metal oxide particles, and soluble metal particles mutually bonded in a dispersed state and a step of performing a developing treatment on the produced particles by the use of the developing solution thereby dissolving out the soluble metal from the particles to give rise to microporous catalyst particles.

### BRIEF DESCRIPTION OF THE DRAWINGS

Other objects, features, and advantages of the invention will become apparent from the following description taken together with the drawings, in which:
Fig. 1 is a schematic magnified perspective view of a catalyst produced by the conventional coprecipitation method;
Fig. 2 is a schematic magnified cross section illustrating one example of the catalyst of the present invention;
Fig. 3 is a graph showing the space-time yields in the reactions for methanol synthesis by using the Cu-ZnO catalysts produced under the conditions of varying times of the mechanical alloying treatment in Example 1 and the Cu-ZnO catalyst of the same composition produced by the coprecipitation method; and
Fig. 4 is a graph showing the changes in catalytic activity with the elapse of reaction time in the reactions for methanol synthesis by using the Cu-ZnO catalyst produced under the condition of 120 hours of the mechanical alloying treatment in Example 1 and the Cu-ZnO catalyst of the same composition produced by the coprecipitation method.

### DETAILED DESCRIPTION OF THE INVENTION

The present invention discloses a technique for producing a catalyst obviating the necessity for a reducing pretreatment, allowing easy handling, and having a structure sparingly inducible of sintering by a simpler process than the conventional method using a metal salt solution as the starting raw material.

To be specific, the present invention has for the primary feature thereof the production of a catalyst by subjecting the mixture of a catalytically active metal and a metal oxide, severally in the form of a powder, granules, or foils, to a mechanical alloying treatment (hereinafter referred to briefly as "MA treatment").

When the mixture of one or more catalytically active metals or alloys thereof and one or more metal oxides is subjected to the MA treatment, the individual particles thereof are ground mechanically into very fine particles and these very fine particles are thoroughly mixed to give rise to catalyst particles 10 of a structure such that catalytically active metal particles 11 are each enclosed with metal oxide particles 12 as illustrated in Fig. 2. The catalyst particles 10, therefore, operate so as to promote the reaction aimed at with very high efficiency and manifest a great catalytic activity because the surfaces of contact between the metal oxide parts as a co-catalyst and the metal parts as the catalytically active sites are numerous and large in area. Further, in the state in which the catalytically active metal particles 11 are each enclosed with the metal oxide particles 12 as described above, the catalyst particles manifest improved thermal stability and sparingly succumb to sintering because the catalytically active metal particles are bonded to the metal oxide particles excelling in resistance to heat, durability, etc. through the strong interaction between them. The preferred condition for enabling the produced catalyst to acquire high thermal stability and high activity is that the MA treatment should continue to impart a mechanical energy until the crystal grains of both the metal and the metal oxide reach particle diameters of not more than 50 nm, preferably not more than 20 nm. Though the duration of the MA treatment for satisfying this requirement cannot be generally defined because it varies with the kind and the particle diameter of the starting raw material, it is generally not less than 20 hours, preferably not less than 60 hours, and particularly preferably not less than 100 hours.

Since the production of the catalyst by the present invention relies on the MA treatment, it is hardly subject to the limit imposed on the combination of component elements of the material unlike the production by the conventional coprecipitation method or impregnation method. As the metal mentioned above, any metal which functions at all as a catalyst for the reaction aimed at may be used. As the metal oxide mentioned above, any metal oxide which functions as a co-catalyst in the catalytic reaction aimed at or manifests resistance to heat may be used. In the production of a catalyst which manifests a high catalytic activity for hydrogenation of carbon dioxide and/or carbon monoxide, for example, it is appropriate to use at least one element selected from among Fe, Co, Ni, Cu, Rh, Pd, Ag, Pt, and Au as the metal and at least one of the oxides of Mg, Al, Si, P, Ca, Sc, Ti, V, Cr, Mn, Fe, Co, Ni, Cu, Zn, Ga, Ge, Sr, Y, Zr, Nb, Mo, Cd, In, Sn, Ba, Hf, Ta, W, Pb, Bi, La, Ce, Pr, Nd, Pm, Sm, Eu, Gd, Tb, Dy, Ho, Er, Tm, Yb, and Lu, particularly at least one oxide selected from among Al₂O₃, SiO₂, TiO₂, Cr₂O₃, ZnO, Ga₂O₃, ZrO₂, Nb₂O₅, CeO₂, and La₂O₃, as the metal oxide.

Generally, the ratio of the metal to the metal oxide in the catalyst particles is properly an equimolar ratio. Depending on the catalytic reaction aimed at and the reaction conditions involved therein, however, the proportion of the metal which is responsible for the catalytic activity may be increased or the proportion of the metal oxide which is excellent in the resistance to heat may be increased. When an easily oxidizable metal is used in the MA treatment which is performed in the air atmosphere, since the proportion of the metal oxide in the produced catalyst tends to be higher than that in the starting raw material, the proportion of the metal oxide in the produced catalyst can be controlled as by increasing the proportion of the metal in the starting raw material. It is permissible to perform the MA treatment in an inert atmosphere, depending on the purpose of the reaction.

The catalyst particles of amply high activity is obtained solely by the MA treatment mentioned above. However, by adding a metal soluble in the developing solution to be used, such as, for example, at least one of such metals as Al, Zn, Si, Pb, Sn, and Mg which are soluble in an aqueous alkali solution, to the raw material, performing the MA treatment on the raw material as described above, and then carrying out the developing treatment or leaching treatment with the treating liquid (developing solution or leaching solution) capable of dissolving the soluble metal mentioned above, the produced catalyst is enabled to acquire a microporous structure and a specific surface area of about 150 m²/g or over and manifest a copiously exalted catalytic activity. The term "developing treatment" or "leaching treatment" used herein means a treatment for dissolving and extracting the soluble metal mentioned above with the developing solution from the catalyst particles combining numerous catalytically active metal particles, metal oxide particles, and soluble metal particles and consequently endowing the catalyst particles with a microporous structure. As the developing solution, therefore, the aqueous solutions of various alkalis, acids, or salts which are capable of dissolving and extracting the soluble metal mentioned above can be used. The developing solution to be selected and used herein should be incapable or sparingly capable of dissolving the other components of the catalyst particles, i.e. the catalytically active metal and the metal oxide.

By combining the MA treatment and the developing treatment as described above, the microporous catalyst particles having metal oxide particles bonded to the peripheries of the catalytically active metal particles and further containing micropores formed by the developing treatment mentioned above are obtained.

Appropriately, the ratio of the catalytically active metal (M¹) to the metal (M²) soluble in the developing solution such as, for example, an alkali-soluble metal (the ratio of the components in the raw material or the ratio thereof in the formed catalyst particles), M¹ : M², is in the range of 50 - 1 : 50 - 99% by weight (wt %). If the proportion of the soluble metal (M²) is less than 50 wt%, the produced catalyst particles will not easily acquire a microporous structure of sufficiently high porosity and will consequently incur difficulty in attaining a conspicuous improvement in catalytic activity. Conversely, if this proportion exceeds 99 wt%, the produced catalyst particles will suffer such an increase in the extracted component as to render the production inefficient and also will be deficient in mechanical strength.

Advantageously, as the metal soluble in the developing solution mentioned above, at least one alkali-soluble metal to be selected from among Al, Zn, Si, Pb, Sn, and Mg is used. As the developing solution to be used herein, it is proper to use the developing solution containing at least one compound selected from among Na₂CO₃, K₂CO₃, NaOH, KOH, Ca(OH)₂, and NH₄OH when one or more elements selected from among Al, Zn, Si, Pb, and Sn is used as the alkali-soluble metal or the developing solution containing at least one compound selected from among Na₂CO₃, K₂CO₃, NaOH, KOH, Ca(OH)₂, NH₄OH, NaCl, and KCl when Mg is used as the alkali-soluble metal.

By the method of the present invention, the metal-metal oxide catalyst manifesting a high activity and sparingly incurring sintering, particularly the catalyst for the reaction of hydrogenation of carbon dioxide and/or carbon monoxide, can be obtained. The method of the present invention is capable of producing the catalyst simply with high reproducibility because it has a small number of steps of process as compared with the conventional method using a metal salt solution as the starting raw material. Particularly, the production of the catalyst resorting solely to the MA treatment and omitting the developing treatment obviates the necessity for the disposal of effluent and allows both the equipment and the operation to be profusely simplified and permits the cost of production of the catalyst to be generously decreased as compared with the conventional method because this operation is a perfectly dry process. Such methods as the coprecipitation method and the impregnation method which use a metal salt solution as the starting raw material, depending on the purpose for which the catalyst is to be used, require a reducing treatment to be performed in a reducing atmosphere such as hydrogen with the temperature of the treatment controlled accurately and necessitate a complicated treatment prior to use because the active metal element incurs a change of state into an oxide during the course of process without fail. In contrast thereto, the method of the present invention for the production of the catalyst is at a prominent advantage in obtaining at the end of the MA treatment or the developing treatment a catalyst formed of a metal and an oxide and, therefore, obviating the necessity for subjecting the produced catalyst to a subsequent calcining treatment or subjecting it to such a complicated pretreatment as the reducing treatment prior to use because it uses a metal and an oxide for the starting raw material.

Further, the technique of the present invention for the production of the catalyst is fully adaptable for the production of catalysts for various reactions such as, for example, other hydrogenations, oxidation, dehalogenation, amination, isomerization, alkylation, dehydrogenation, and polymerizations.

Now, the present invention will be more specifically described below with reference to working examples and comparative examples.

### Example 1:

Varying species of catalyst particles were produced by subjecting a mixed powder of Cu and ZnO (Cu : ZnO = 50 : 50 wt%) to the MA treatment in a ball mill made of alumina, with the treating time varied.

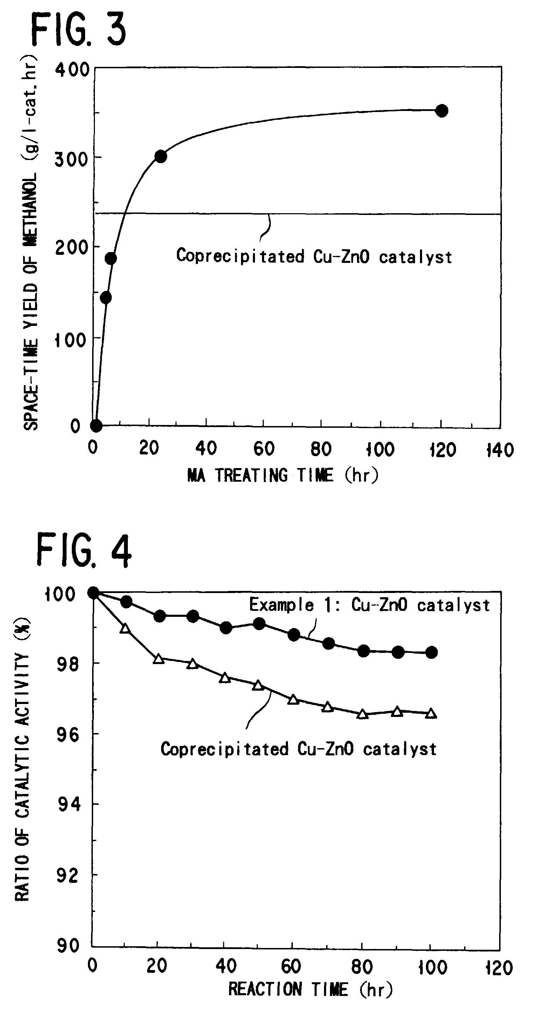

Each species of the catalyst particles thus obtained were tested for catalyst properties relating to the hydrogenation of carbon dioxide by the use of a pressurised fixed-bed flow type reactor by passing therethrough a H₂/CO₂ mixed gas (H₂ : CO₂ = 3 : 1) at a spatial velocity, SV, of 26000/hr under the conditions of a reaction temperature of 250 °C and a reaction pressure of 5 MPa, and analyzing the product by on-line gas chromatography to determine the catalytic activity in the synthesis of methanol from carbon dioxide [space-time yield of methanol : the yield of methanol (g) per unit volume (1 liter) and unit reaction time (1 hr)]. The results are shown in Fig. 3. For comparison, the results similarly obtained of the Cu-ZnO catalyst of the same composition produced by the conventional coprecipitation method are additionally shown in Fig. 3.

The durability (change of catalytic activity along the course of the reaction time) of the catalyst of Example 1 and that of the Cu-ZnO catalyst of the same composition produced by the coprecipitation method are compared in Fig. 4.

The space-time yields of methanol obtained of the catalyst produced by performing the MA treatment for 120 hours according to the present invention and the catalyst of the same composition produced by the conventional coprecipitation method are shown in Table 1 below.

**Table 1**

| Example No. | Catalyst | Method of production | Space-time yield of methanol (g/l-cat·hr) |
|---|---|---|---|
| Example 1 | Cu/ZnO (50/50 wt%) | 120 hours' MA treatment | 360 |
| Comparative Example 1 | Cu/ZnO (50/50 wt%) | Coprecipitation method | 235 |

It is clearly noted from the results shown in Fig. 3 that among the catalysts produced by the method of the present invention, that resulting from about 11 hours' MA treatment obtained a methanol yield equal to that obtained by the catalyst produced by the conventional coprecipitation method and that resulting from 120 hours' MA treatment obtained a methanol yield of more than 1.5 times that obtained by the catalyst produced by the coprecipitation method.

From the results shown in Fig. 4, it is noted that the deterioration of the catalytic activity of the catalysts produced by the method of the present invention due to the elapse of the reaction time was low compared with the catalysts obtained by the conventional coprecipitation method.

### Examples 2 through 10:

Varying species of catalyst particles were produced in consequence of 120 hours' MA treatment by following the procedure of Example 1 while using the metal oxides shown in Table 2 instead.

The catalysts were tested for catalytic activity (space-time yield of methanol) in the synthesis of methanol from carbon dioxide in the same manner as in Example 1. The results are shown in Table 2 below.

**Table 2**

| Example No. | Catalyst | Time of MA treatment | Space-time yield of methanol (g/l-cat·hr) |
|---|---|---|---|
| 2 | Cu/SiO₂ (50/50 wt%) | 120hr | 70 |
| 3 | Cu/Al₂O₃ (50/50 wt%) | 120hr | 80 |
| 4 | Cu/TiO₂ (50/50 wt%) | 120hr | 120 |
| 5 | Cu/ZrO₂ (50/50 wt%) | 120hr | 125 |
| 6 | Cu/CeO₂ (50/50 wt%) | 120hr | 180 |
| 7 | Cu/Cr₂O₃ (50/50 wt%) | 120hr | 95 |
| 8 | Cu/Ga₂O₃ (50/50 wt%) | 120hr | 200 |
| 9 | Cu/Nb₂O₅ (50/50 wt%) | 120hr | 170 |
| 10 | Cu/La₂O₃ (50/50 wt%) | 120hr | 170 |

### Examples 11 through 15:

Varying species of catalyst particles were produced by the MA treatment by following the procedure of Example 1 while using the catalytically active metals shown in Table 3 instead and changing the time of MA treatment to the durations shown in Table 3.

The catalysts were tested for catalytic activity (space-time yield of methanol) in the synthesis of methanol from carbon dioxide in the same manner as in Example 1. The results are shown in Table 3 below.

**Table 3**

| Example No. | Catalyst | Time of MA treatment | Space-time yield of methanol (g/l-cat·hr) |
|---|---|---|---|
| 11 | Au/ZnO (50/50 wt%) | 100hr | 200 |
| 12 | Ag/ZnO (50/50 wt%) | 100hr | 170 |
| 13 | Pb/ZnO (50/50 wt%) | 200hr | 300 |
| 14 | Pt/ZnO (50/50 wt%) | 200hr | 250 |
| 15 | Rh/ZnO (50/50 wt%) | 200hr | 200 |

It is clearly noted by comparing Table 2 and Table 3 given above that as the catalyst for the synthesis of methanol, the systems using ZnO, CeO₂, Ga₂O₃, Nb₂O₅, La₂O₃, and particularly ZnO as metal oxides were effective and that among other various metals usable as catalytically active metals, Cu and Pb manifested particularly high catalytic activity.

### Examples 16 through 18:

Varying species of catalyst particles were produced by the MA treatment performed for 120 hours by following the procedure of Example 1 while using the two kinds of metal oxides shown in Table 4 instead.

The catalysts were tested for catalytic activity (space-time yield of methanol) in the synthesis of methanol from carbon dioxide in the same manner as in Example 1. The results are shown in Table 4 below. For comparison, the results similarly obtained of the catalysts of the same composition produced by the conventional coprecipitation method are additionally shown in Table 4.

**Table 4**

| Example No. | Catalyst | Method of production | Space-time yield of methanol (g/l-cat·hr) |
|---|---|---|---|
| Example 16 | Cu/ZnO/Al₃0₃ (50/45/5 wt%) | 120 hours' MA treatment | 550 |
| Example 17 | Cu/ZnO/Ga₂0₃ (50/25/25 wt%) | 120 hours' MA treatment | 580 |
| Example 18 | Cu/ZnO/Ga₂0₃ (50/30/20 wt%) | 120 hours' MA treatment | 570 |
| Comparative Example 2 | Cu/ZnO/Al₂0₃ (50/45/5 wt%) | Coprecipitation method | 385 |
| Comparative Example 3 | Cu/ZnO/Ga₂0₃ (50/25/25 wt%) | Coprecipitation method | 400 |
| Comparative Example 4 | Cu/ZnO/Ga₂0₃ (50/30/20 wt%) | Coprecipitation method | 400 |

It is clearly noted by comparing Table 1 and Table 4 given above that for the synthesis of methanol, the catalysts using two kinds of metal oxides showed extremely high catalytic activity.

### Examples 19 through 24:

Varying species of catalyst particles were produced by the MA treatment performed for 120 hours by following the procedure of Examples 16 through 18 while using the catalytically active metals shown in Table 5 instead.

The catalysts were tested for catalytic activity (space-time yield of methanol) in the synthesis of methanol from carbon dioxide in the same manner as in Example 1. The results are shown in Table 5 below.

**Table 5**

| Example No. | Catalyst | Time of MA treatment | Space-time yield of methanol (g/l-cat·hr) |
|---|---|---|---|
| 19 | Au/ZnO/Al₂O₃ (50/45/5 wt%) | 120hr | 350 |
| 20 | Au/ZnO/Ga₂O₃ (50/25/25 wt%) | 120hr | 370 |
| 21 | Au/ZnO/Ga₂O₃ (50/30/20 wt%) | 120hr | 360 |
| 22 | Ag/ZnO/Al₂O₃ (50/45/5 wt%) | 120hr | 320 |
| 23 | Ag/ZnO/Ga₂O₃ (50/25/25 wt%) | 120hr | 310 |
| 24 | Ag/ZnO/Ga₂O₃ (50/30/20 wt%) | 120hr | 300 |

### Examples 25 through 27:

Varying species of catalyst particles were produced by the MA treatment performed for 120 hours by following the procedure of Example 1 while using the two kinds of catalytically active metals and the two kinds of metal oxides shown in Table 6 instead.

The catalysts were tested for catalytic activity (space-time yield of methanol) in the synthesis of methanol from carbon dioxide in the same manner as in Example 1. The results are shown in Table 6 below.

**Table 6**

| Example No. | Catalyst | Time of MA treatment | Space-time yield of methanol (g/l-cat·hr) |
|---|---|---|---|
| 25 | Cu/Ni/ZnO/Al₂O₃ (45/5/45/5 wt%) | 120hr | 560 |
| 26 | Cu/Co/ZnO/Ga₂O₃ (45/5/25/25 wt%) | 120hr | 600 |
| 27 | Cu/Fe/ZnO/Ga₂O₃ (45/5/30/20 wt%) | 120hr | 590 |

It is clearly noted by comparing Table 6 with Tables 4 and 5 given above that among other systems using the two kinds of metal oxides, those using Cu as a catalytically active metal showed conspicuous catalytic activity and that even among the systems using Cu of such highly catalytic activity, those using other catalytically active metal in combination enjoyed improvement in catalytic activity.

### Example 28:

Microporous catalyst particles were produced by subjecting a mixed powder of Cu, ZnO, and Al (Cu : ZnO : Al = 25 : 25 : 50 wt%) to 120 hours' MA treatment in a ball mill made of alumina and subsequently subjecting the product of MA treatment to a developing treatment in an aqueous 20 wt% NaOH solution kept at 50°C .

The catalyst consequently obtained was tested for catalyst properties relating to the hydrogenation of carbon dioxide. The catalyst properties which were determined by the same fixed bed pressure flow type reaction apparatus as used in Example 1 were rated under the same conditions. The results are shown in Table 7.

### Examples 29 through 32:

Various species of microporous catalyst particles were produced by the MA treatment performed for 120 hours and the subsequent developing treatment by following the procedure of Example 28 while using the metal oxides and the alkali-soluble metals shown in Table 7 instead.

The catalysts consequently obtained were tested for catalytic activity relating to the hydrogenation of carbon dioxide in the same manner as in Example 28. The results are shown additionally in Table 7.

### Examples 33 through 35:

Various species of microporous catalyst particles were produced by performing the MA treatment and the subsequent developing treatment by following the procedure of Example 28 while using the catalytically active metals shown in Table 7 and changing the time of MA treatment to the durations shown in Table 7 instead.

The catalysts consequently obtained were tested for catalytic activity relating to the hydrogenation of carbon dioxide in the same manner as in Example 28. The results are shown additionally in Table 7.

### Examples 36 through 38:

Various species of microporous catalyst particles were produced by performing the MA treatment for 120 hours and the subsequent developing treatment by following the procedure of Example 28 while using the two kinds of metal oxides shown in Table 7 and the catalytically active metals and the alkali-soluble metals shown in Table 7 instead.

The catalysts consequently obtained were tested for catalytic activity relating to the hydrogenation of carbon dioxide in the same manner as in Example 28. The results are shown additionally in Table 7.

**Table 7**

| Example No. | Catalyst | Time of MA treatment | Conditions for development | Space-time yield of methanol (g/l-cat·hr) |
|---|---|---|---|---|
| 28 | Cu/ZnO/Al (5/5/90 wt%) | 120hr | 20%NaOH | 540 |
| 29 | Cu/SiO₂/Al (5/5/90 wt%) | 120hr | 20%NaOH | 100 |
| 30 | Cu/Al₂O₃/Mg (5/5/90 wt%) | 120hr | 20%NaCl | 140 |
| 31 | Cu/TiO₂/Si (10/10/80 wt%) | 120hr | 20%NaOH | 190 |
| 32 | Cu/ZrO₂/Al (10/10/80 wt%) | 120hr | 20%NaOH | 175 |
| 33 | Au/ZnO/Al (5/5/90 wt%) | 120hr | 20%NaOH | 270 |
| 34 | Ag/ZnO/Al (5/5/90 wt%) | 100hr | 20%NaOH | 210 |
| 35 | Pd/ZnO/Al (5/5/90 wt%) | 200hr | 20%NaOH | 450 |
| 36 | Cu/ZnO/Al₂O₃/Al (25/22.5/2.5/50 wt%) | 120hr | 20%KOH | 650 |
| 37 | Cu/ZnO/Ga₂O₃/Si (10/5/5/80 wt%) | 120hr | 20%KOH | 670 |
| 38 | Au/ZnO/Al₂O₃/Al (10/9/1/80 wt%) | 120hr | 20%NaOH | 450 |

It is clearly noted by comparing Table 7 with Tables 1 through 5 given above that the microporous catalysts obtained by performing the developing treatment subsequently to the MA treatment enjoyed a notable improvement in catalytic activity.

While certain specific working examples have been disclosed herein, the invention may be embodied in other specific forms without departing from the spirit or essential characteristics thereof. The described examples are therefore to be considered in all respects as illustrative and not restrictive, the scope of the invention being indicated by the appended claims rather than by the foregoing description and all changes which come within the meaning and range of equivalency of the claims are, therefore, intended to be embraced therein.

## Claims

1. A metal-metal oxide catalyst comprising catalyst particles each having a plurality of catalytically active metal particles and metal oxide particles mutually bonded in a dispersed state.

2. The catalyst according to claim 1, which has a microporous structure.

3. The catalyst according to claim 1 or 2, wherein said catalytically active metal is at least one metal selected from the group consisting of Fe, Co, Ni, Cu, Rh, Pd, Ag, Pt, and Au and said metal oxide is at least one metal oxide selected from the group consisting of the oxides of Mg, Al, Si, P, Ca, Sc, Ti, V, Cr, Mn, Fe, Co, Ni, Cu, Zn, Ga, Ge, Sr, Y, Zr, Nb, Mo, Cd, In, Sn, Ba, Hf, Ta, W, Pb, Bi, La, Ce, Pr, Nd, Pm, Sm, Eu, Gd, Tb, Dy, Ho, Er, Tm, Yb, and Lu.

4. The catalyst according to any one of claims 1 to 3, wherein said catalyst particle is composed of crystal grains of said catalytically active metal and of said metal oxide having a size not more than 50 nm.

5. The catalyst according to any one of claims 1 to 4, which is a catalyst for hydrogenation of carbon oxide.

6. A method for the production of a metal-metal oxide catalyst, which comprises subjecting a mixture of a catalytically active metal and a metal oxide, severally in the form of a powder, granules, or foils, to a mechanical alloying treatment thereby forming catalyst particles each having a plurality of catalytically active metal particles and metal oxide particles mutually bonded in a dispersed state.

7. A method for the production of a metal-metal oxide catalyst, which comprises the steps of:
subjecting a mixture of a catalytically active metal, a metal oxide, and a metal soluble in a developing solution, severally in the form of a powder, granules, or foils, to a mechanical alloying treatment, thereby producing particles each having a plurality of catalytically active metal particles, metal oxide particles, and soluble metal particles mutually bonded in a dispersed state and
subjecting said produced particles to a developing treatment with the developing solution thereby dissolving out the soluble metal from the particles to give rise to microporous catalyst particles.

8. The method according to claim 6 or 7, wherein said catalytically active metal is at least one metal selected from the group consisting of Fe, Co, Ni, Cu, Rh, Pd, Ag, Pt, and Au and said metal oxide is at least one metal oxide selected from the group consisting of the oxides of Mg, Al, Si, P, Ca, Sc, Ti, V, Cr, Mn, Fe, Co, Ni, Cu, Zn, Ga, Ge, Sr, Y, Zr, Nb, Mo, Cd, In, Sn, Ba, Hf, Ta, W, Pb, Bi, La, Ce, Pr, Nd, Pm, Sm, Eu, Gd, Tb, Dy, Ho, Er, Tm, Yb, and Lu.

9. The method according to claim 7 or 8, wherein said metal soluble in the developing solution is at least one alkali-soluble metal selected from the group consisting of Al, Zn, Si, Pb, Sn, and Mg and the mixing ratio of said catalytically active metal to said alkali-soluble metal is in the range of 50 : 50 to 1 : 99% by weight.

10. The method according to any one of claims 7 to 9, wherein said alkali-soluble metal is at least one member selected from the group consisting of Al, Zn, Si, Pb, and Sn and said developing treatment is carried out with a developing solution containing at least one compound selected from the group consisting of Na₂CO₃, K₂CO₃, NaOH, KOH, Ca(OH)₂, and NH₄OH.

11. The method according to any one of claims 7 to 9, wherein said alkali-soluble metal is magnesium and said developing treatment is carried out with a developing solution containing at least one compound selected from the group consisting of Na₂CO₃, K₂CO₃, NaOH, KOH, Ca(OH)₂, NH₄OH, NaCl, and KCl.
